# EUROPEAN PATENT SPECIFICATION

(11) **EP 3 470 844 B1**
(45) Date of publication and mention of the grant of the patent: **01.09.2021**
(21) Application number: 17813494.6
(22) Date of filing: 24.05.2017
(51) Int. Cl.: G01N 33/574, G01N 30/88, G01N 30/72

(54) **METHOD FOR PROVIDING DIAGNOSTIC INFORMATION FOR BILIARY TRACT CANCER AND APPARATUS FOR DIAGNOSING BILIARY TRACT CANCER**
VERFAHREN ZUR BEREITSTELLUNG VON DIAGNOSTISCHEN INFORMATIONEN FÜR GALLENGANGKARZINOM UND VORRICHTUNG ZUR DIAGNOSE VON GALLENGANGKARZINOM
PROCÉDÉ PERMETTANT DE FOURNIR DES INFORMATIONS DE DIAGNOSTIC POUR LE CANCER DU TRACTUS BILIAIRE ET APPAREIL DE DIAGNOSTIC DU CANCER DU TRACTUS BILIAIRE

(30) Priority: 13.06.2016 KR 20160073340
(43) Date of publication of application: 17.04.2019
(73) Proprietor: National Cancer Center, Goyang-si, Gyeonggi-do 10408 (KR)
(72) Inventor: YOO, Byong Chul, Goyang-si Gyeonggi-do 10415 (KR); KIM, Kyung Hee, Seoul 06560 (KR); WOO, Sang Myung, Seoul 06289 (KR); KONG, Sun Young, Goyang-si Gyeonggi-do 10415 (KR); KIM, Tae Hyun, Seoul 06284 (KR); PARK, Sang Jae, Goyang-si Gyeonggi-do 10374 (KR); LEE, Woo Jin, Seoul 06284 (KR); HAN, Sung-Sik, Seoul 06506 (KR)
(74) Representative: Shearman, James Ward
(86) International application number: PCT/KR2017/005394
(87) International publication number: WO 2017/217669

(56) References cited:
- EP-A1- 2 623 984
- JP-A- 2012 237 685
- KR-A- 20050 099 581
- KR-A- 20130 010 831
- KR-A- 20130 079 986
- KUHN, T. ET AL.: 'Higher Plasma Levels of Lysophosphat Idylcholine 18:0 are Related to a Lower Risk of Common Cancers in a Prospective Metabolomics Study' BMC MEDICINE vol. 14, no. 13, 28 January 2016, pages 1 - 9, XP055449129

## Description

### TECHNICAL FIELD

The present disclosure relates to a method for providing diagnostic information for diagnosing biliary tract cancer and an apparatus for diagnosing biliary tract cancer.

### BACKGROUND

Cancer is a disease in which functions of normal cells are hindered by indefinite proliferation of cells. Representative examples of cancer include lung cancer, gastric cancer (GC), breast cancer (BRC), colorectal cancer (CRC), biliary tract cancer, and ovarian cancer (OVC), and so on; however, cancer can occur virtually in any tissues.

A lot of efforts have been made for early diagnosis of cancer, and for biliary tract cancers, there are abdominal CT, CA19-9 Electrochemiluminescence immunoassay (ECLIA), Alpha-fetoprotein test, and so on. However, a diagnosis method that has a high level of accuracy and is noninvasive has not been developed yet.

Korean patent application KR 10-2013-0010831 describes biomarkers for biliary tract cancer.

### DETAILED DESCRIPTION OF THE INVENTION

### TECHNICAL PROBLEM

An aspect of the present disclosure is directed to providing a method for providing diagnostic information for diagnosing biliary tract cancer and an apparatus for diagnosing biliary tract cancer.

### SOLUTION TO PROBLEM

According to an aspect of the present disclosure, there is provided a method for providing diagnostic information for diagnosing biliary tract cancer including measuring a concentration of a marker for predicting biliary tract cancer in previously obtained biological samples including whole blood; and providing diagnostic information for biliary tract cancer using the measured concentration of the marker. The marker includes Nudifloramide.

The marker may further include at least one of LPC 18:0 and fibrinogen alpha chain.

Criterion concentration of the Nudifloramide for biliary tract cancer diagnosis may be 220 pg/µℓ to 320 pg/µℓ.

Criterion concentration of the fibrinogen alpha chain may be 130 pg/µℓ to 200 pg/µℓ.

Concentration of the LPC 18:0 may be measured using a mass spectrometer, and criterion concentration of the LPC 18:0 for biliary tract cancer diagnosis may be 1,500,000 au to 2,000,000 au.

In the step of providing information, determination of whether or not biliary tract cancer may be made based on a certain concentration of Nudifloramide and fibrinogen alpha chain or higher and a certain concentration of the LPC 18:0 or less.

The biological samples may further include serum.

The concentration may be measured by mass spectrometry.

According to an aspect of the present disclosure, there is provided an apparatus for diagnosing biliary tract cancer including an input unit configured to input mass spectrum data detected in biological samples; and a diagnosis unit configured to calculate concentration of a marker for prediction of biliary tract cancer from the mass spectrum data and determine diagnostic information for biliary tract cancer on basis of the calculated concentration. The marker includes Nudifloramide.

The marker may further include at least one of LPC 18:0 and fibrinogen alpha chain.

Criterion concentration of the Nudifloramide for biliary tract cancer diagnosis may be 220 pg/µℓ to 320 pg/µℓ.

### EFFECTS OF INVENTION

A method for providing diagnostic information for diagnosing biliary tract cancer and an apparatus for diagnosing biliary tract cancer according to embodiments of the present disclosure may have a high level of accuracy and be noninvasive.

### BRIEF DESCRIPTION OF THE DRAWINGS

FIG. 1 illustrates an apparatus for diagnosing biliary tract cancer according to an embodiment of the present disclosure.
FIG. 2 shows a diagnosis result using concentration of Nudifloramide.
FIG. 3 shows a diagnosis result using concentration of LPC 18:0.
FIG. 4 shows another diagnosis result using concentration of LPC 18:0.
FIG. 5 shows a diagnosis result using concentration of fibrinogen alpha chain.

### MODES FOR CARRYING OUT THE INVENTION

In the present disclosure, the term "biological samples" includes samples such as whole blood, serum, and plasma.

The present disclosure is based on that Nudifloramide has been found to be useful as a marker of biliary tract cancer.

The formal title of Nudifloramide is N-Methyl-2-pyridoxone-5-carboxamide; 1,6-Dihydro-1-methyl-6-oxonicotinamide; 3-Carbamoyl-1-methyl-6-pyridone, and the structural formula is the following:

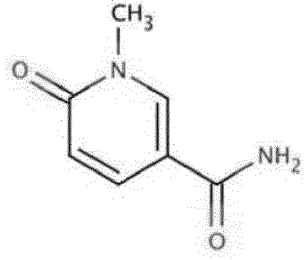

As the marker of biliary tract cancer, lysophosphatidylcholine (LPC) 18:0 and/or fibrinogen alpha chain (FAC) may be further used.

Biological samples, for example, a concentration of a marker in serum (concentration value), is measured, and diagnosis as to whether it is biliary tract cancer can be made from the measured concentration. The measurement of concentration, which is not limited to the following however, may be performed using a mass spectrometer, and concentrations of each marker are measured using arbitrary unit ("au") values which correspond to markers measured by the mass spectrometer.

LPC 18:0 and fibrinogen alpha chain (FAC) serves of improvement of specificity, positive prediction value (PPV) and/or negative prediction value (NPV) of diagnosis result.

In the diagnosis of biliary tract cancer, regarding Nudifloramide and FAC, it is determined as biliary tract cancer if concentrations of Nudifloramide and FAC are higher than or equal to each certain concentration, and regarding LPC 18:0, it is determined as biliary tract cancer if concentration of LPC 18:0 is less than or equal to a certain concentration.

Regarding Nudifloramide, the certain concentration that is a criterion of biliary tract cancer diagnosis may be in range of 150 pg/µℓ to 500 pg/µℓ, 200 pg/µℓ to 350 pg/µℓ, 220 pg/µℓ to 320 pg/µℓ, or 250 pg/µℓ to 300 pg/µℓ.

Regarding FAC, the certain concentration that is a criterion of biliary tract cancer may be in range of 100 pg/µℓ to 250 pg/µℓ, 130 pg/µℓ to 200 pg/µℓ, or 150 pg/µℓ to 180 pg/µℓ.

Regarding LPC 18:0, the certain concentration that is a criterion of biliary tract cancer is au value of mass spectrometer and may be in range of 1,000,000 to 2,500,000, 1,500,000 to 2,000,000, or 1,600,000 to 1,800,000.

Meanwhile, the ranges of the certain concentration above are values under conditions according to experimental examples of the present disclosure. If a specific concentration range is out of the above-mentioned range using other sampling conditions, pretreatment conditions, or other equipment but if it will be within the range of a certain concentration by following the conditions of the present disclosure, it should be understood that it belongs to the scope of right of the present disclosure.

The following will be described in detail with reference to the drawings.

The accompanying drawings are merely illustrations to provide more details of the technical ideas of the present description.

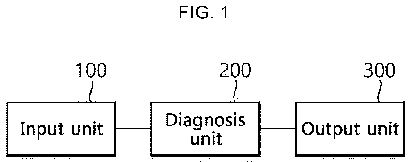

FIG. 1 illustrates an apparatus for diagnosing biliary tract cancer according to embodiments of the present disclosure.

An input unit 100 inputs mass spectrum data detected in biological samples (hereinafter referred to as "diagnosis target data").

A diagnosis unit 200 measures concentration of a marker from diagnosis target data and generates diagnostic information for biliary tract cancer based on the measured concentration. That is, the diagnosis unit 200 determines the biliary tract cancer positive or negative with respect to diagnosis target data. In this process, the diagnosis unit 200 may use Nudifloramide concentration alone or further use at least one of concentrations of LPC 18:0 and fibrinogen alpha chain.

In the diagnosis unit 200, criterion concentrations have been set for respective markers, and criterion concentrations may be changed depending on various information of a diagnosis target person, such as age, gender, whether to have other cancers, and so on.

An output unit 300 may output diagnostic information for biliary tract cancer and use a display.

The present disclosure will be explained in more detail through experimental examples below.

### Obtaining serum

Serum was obtained from 92 patients with biliary tract cancer, 34 patients with pancreatic cancer, 100 patients with lung cancer, 30 patients with gastric cancer, 3 patients with ovarian cancer, and a normal control group of 340 people.

### Extracting serum

Serum of patients with biliary tract caner, patients with other types of cancers, and a normal control group was extracted using modified Bligh and Dyer method. The detailed method is described in the below.

After 1 mℓ of distilled water was added to 50 *µ*ℓ of serum, 2 mℓ of methanol and 0.9 mℓ of dichloromethane were added.

After mixing well, it was left on ice for 30 minutes, and then 1 mℓ of distilled water and 0.9 mℓ of dichloromethane were added.

After that, centrifugation was performed at 1,500 rpm for 10 minutes at room temperature and supernatant was separated, and dried with nitrogen gas.

### Quantitative analysis (concentration measurement)

Extraction sample 5 *µ*ℓ was injected to Nexera X2 LC system (Shimadzu) and a marker was separated using concentration gradient of a solvent to be analyzed (solvent A, 0.1% FA in water; solvent B, 100% ACN; with 1% solvent B for 1.5 min, 1 to 25% B for 4.5 min, 25 to 45% B for 2 min, 45 to 90% B for 2 min, 90% B for 4 min, 90 to 1% B for 0.5 min and 5.5 min in 1% B), and then a quantitative analysis was performed using Triple TOF 5600+ system (SCIEX) mass spectrometer in positive ion MRM mode.

### Nudifloramide

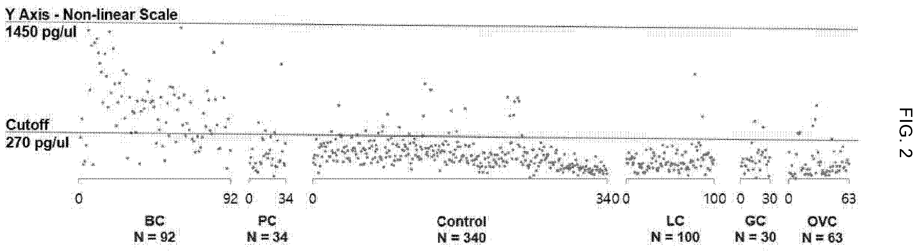

Concentration distribution of Nudifloramide in respective cancers and normal controls are as in FIG. 2, and a determination result based on 270 pg/µℓ is shown in Table 1.

**(Table 1)**

| | Biliary tract cancer (BC) | Pancreatic cancer (PC) | Normal controls | Lung cancer (LC) | Gastric cancer (GC) | Ovarian cancer (OVC) |
|---|---|---|---|---|---|---|
| Nudifloramide270 pg/µℓ over | 74 | 6 | 30 | 2 | 2 | 7 |
| Nudifloramide270 pg/µℓ less | 18 | 28 | 310 | 98 | 28 | 56 |

Based on the determination result in Table 1, the sensitivity, specificity, PPV, and NPV of biliary cancer based on 270 pg/µℓ of Nudifloramide are as follows:
Sensitivity : 80.43% (74/92)
Specificity : 91.71% ((28+310+98+28+56)/(34+340+100+30+63))
Positive Prediction Value (PPV): 61.16% (74/(74+6+30+2+2+7))
Negative Prediction Value (NPV) 96.65% ((28+310+98+28+56)/(18+28+310+98+28+56))

These results indicate that the use of Nudifloramide alone enables high sensitivity, specificity, PPV, and NPV.

### LPC 18:0

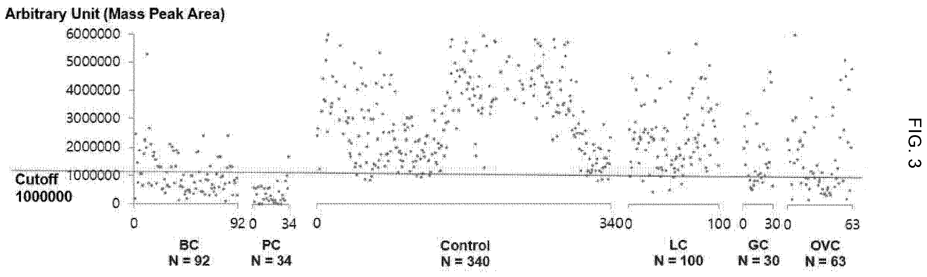

The concentration distribution of LPC 18:0 of each cancer and normal controls are as in FIG. 3, and determination results based on mass spectrometer au 1,000,000 are shown in Table 2.

**(Table 2)**

| | Biliary tract cancer (BC) | Pancreatic cancer (PC) | Normal controls | Lung cancer (LC) | Gastric cancer (GC) | Ovarian cancer (OVC) |
|---|---|---|---|---|---|---|
| LPC 18:0 1,000,000 over | 39 | 1 | 329 | 92 | 17 | 36 |
| LPC 18:0 1,000,000 less | 53 | 33 | 11 | 8 | 13 | 27 |

Based on the determination results of Table 2, the sensitivity, specificity, PPV, and NPV of pancreatic cancer based on mass spectrometer 1,000,000 au of LPC 18:0 are as follows:
Sensitivity: 97.06% (33/34)
Specificity: 82.08% ((39+329+92+17+36)/(92+340+100+30+63))
Positive Prediction Value (PPV) : 22.76% (33/(53+33+11+8+13+27))
Negative Prediction Value (NPV) : 99.81% (1/(39+1+329+92+17+36))

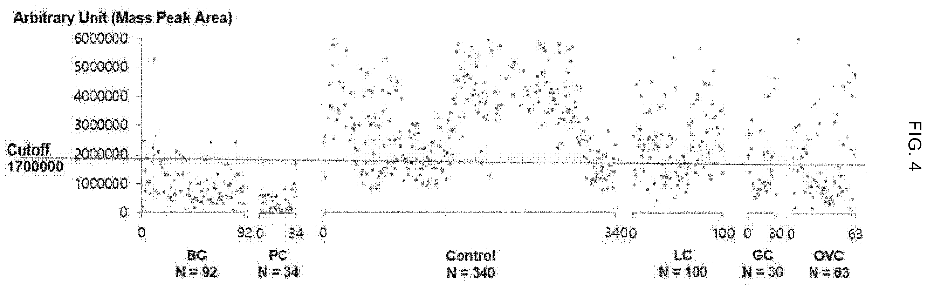

The results obtained when changing the criterion of LPC 18:0 to mass spectrometer 1,700,000 au are shown in FIG. 4 and Table 3.

**(Table 3)**

| | Biliary tract cancer (BC) | Pancreatic caner (PC) | Normal controls | Lung cancer (LC) | Gastric cancer (GC) | Ovarian cancer (OVC) |
|---|---|---|---|---|---|---|
| LPC 18:0 1,700,000 over | 18 | 0 | 266 | 64 | 11 | 25 |
| LPC 18:0 1,700,000 less | 74 | 34 | 74 | 36 | 19 | 38 |

Based on determination result of Table 3, the sensitivity, specificity, PPV, and NPV of pancreatic cancer based on mass spectrometer 1,700,000 au of LPC 18:0 are as follows:
Sensitivity : 100% (34/34)
Specificity : 61.44% ((18+266+64+11+25)/(92+340+100+30+63))
Positive Prediction Value (PPV) : 12.36% (34/(74+34+74+36+19+38))
Negative Prediction Value (NPV) 100% ((18+266+64+11+25)/(18+0+266+64+11+25))

From the above results, it can be seen that LPC 18:0 is a useful marker for pancreatic cancer. In addition, when criterion concentration of LPC 18:0 is high, it can be used as a marker for not only pancreatic cancer but also biliary tract cancer as indicated in the below.

### Nudifloramide + LPC 18:0

The determination results considering the concentration of both two markers by setting the concentration criterion of Nudifloramide as 270 pg/µℓ and setting the concentration criterion of LPC 18:0 as mass spectrometer 1,700,000 au are shown in Table 4. In the below table, "YES" means a case which meets both the condition of less than Nudifloramide 270 pg/µℓ and the condition of LPC 18:0, and "NO" means a case which meets neither of two conditions.

**(Table 4)**

| | Biliary tract cancer (BC) | Pancreatic caner (PC) | Normal controls | Lung cancer (LC) | Gastric cancer (GC) | Ovarian cancer (OVC) |
|---|---|---|---|---|---|---|
| YES | 58 | 6 | 3 | 1 | 0 | 5 |
| NO | 34 | 28 | 337 | 99 | 30 | 58 |

The determination results of Table 4 shows that the sensitivity, specificity, PPV, and NPV of biliary tract cancer less than Nudifloramide 270pg/µℓ and over LPC 18:0 1,700,000 are as follows:
Sensitivity : 63.04% (58/92)
Specificity : 97.35% (28+337+99+30+58)/(34+340+100+30+63))
Positive Prediction Value (PPV) : 79.45% (58/(58+6+3+1+0+5))
Negative Prediction Value (NPV) 94.18% ((28+337+99+30+58)/(34+28+337+99+30+58))

When considering both Nudifloramide and LPC 18:0 as indicated in the above results, sensitivity of biliary tract cancer screening is somewhat reduced, but specificity, PPV, and NPV are all increased for clinical use.

### FAC

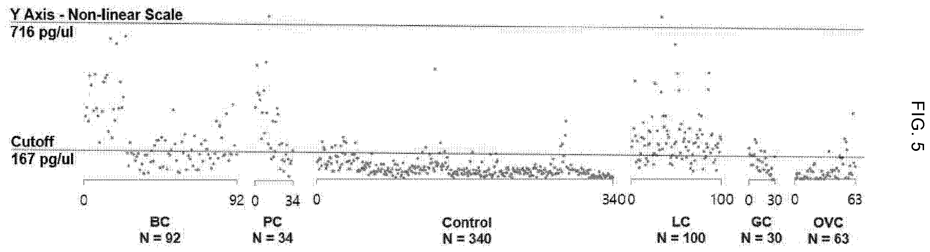

The concentration distribution of FAC in each cancer and normal controls is shown in FIG. 5, and determination results based on 167 pg/µℓ are shown in Table 5.

**(Table 5)**

| | Biliary tract cancer (BC) | Pancreatic caner (PC) | Normal controls | Lung cancer (LC) | Gastric cancer (GC) | Ovarian cancer (OVC) |
|---|---|---|---|---|---|---|
| FAC 167pg/µℓ over | 49 | 23 | 19 | 66 | 5 | 5 |
| FAC 167pg/µℓ less | 43 | 11 | 321 | 34 | 25 | 58 |

Based on the determination results in Table 5, sensitivity, specificity, PPV, and NPV of biliary tract cancer based on FAC 167 pg/µℓ are as follows:
Sensitivity : 53.26% (49/92)
Specificity : 79.18% ((11+321+34+25+58)/(34+340+100+30+63))
Positive Prediction Value (PPV) : 29.34% (49/(49+23+19+66+5+5))
Negative Prediction Value (NPV) ((11+321+34+25+58)/(43+11+321+34+25+58))

### Nudifloramide + LPC 18:0 + FAC

When considering all of Nudifloramide, LPC 18:0, and FAC, sensitivity, specificity, PPV, and NPV are shown in Table 6, where the criterion concentrations are Nudifloramide 270 pg/µℓ, LPC 18:0 1,700,000 au, and FAC 167 pg/µℓ.

**(Table 6)**

| | | TRUE | | |
|---|---|---|---|---|
| | | Biliary tract cancer | Non-biliary tract cancer | |
| Predicted | Biliary tract cancer | 70 | 27 | PPV 72.16% |
| | Non-biliary tract cancer | 22 | 540 | NPV 96.09% |
| | | Sensitivity 76.09% | Specificity 95.24% | |

When considering all of Nudifloramide, LPC 18:0, and FAC as indicated in the above results, sensitivity of biliary tract cancer screening is somewhat reduced, but specificity, PPV, and NPV are all increased for clinical use.

The above-described embodiments are illustrative of the present description. It will be understood by those skilled in the art that various changes in form and details may be made therein without departing from the scope of the invention as defined by the appended claims.

## Claims

1. A method for providing diagnostic information for diagnosing biliary tract cancer comprising:
measuring a concentration of a marker for predicting biliary tract cancer in previously obtained biological samples including whole blood; and
providing diagnostic information for biliary tract cancer using the measured concentration of the marker,
wherein the marker comprises Nudifloramide.

2. The method of claim 1, wherein the marker further comprises at least one of LPC 18:0 and fibrinogen alpha chain.

3. The method of claim 1 or claim 2, wherein criterion concentration of the Nudifloramide for biliary tract cancer diagnosis is 220 pg/µℓ to 320 pg/µℓ.

4. The method of claim 2, wherein criterion concentration of the fibrinogen alpha chain is 130 pg/µℓ to 200 pg/µℓ.

5. The method of claim 2, wherein concentration of the LPC 18:0 is measured using a mass spectrometer, and criterion concentration of the LPC 18:0 for biliary tract cancer diagnosis is 1,500,000 au to 2,000,000 au.

6. The method of claim 2, the provision of the information is determination of whether or not biliary tract cancer based on a certain concentration of the Nudifloramide and the fibrinogen alpha chain or higher and a certain concentration of the LPC 18:0 or less.

7. The method of claim 1, wherein the biological samples further comprise serum

8. The method of claim 1, wherein the concentration is measured by mass spectrometry.

9. An apparatus for diagnosing biliary tract cancer comprising:
an input unit configured to input mass spectrum data detected from biological samples; and
a diagnosis unit configured to calculate concentration of a marker for prediction of biliary tract cancer from the mass spectrum data and determine diagnostic information for biliary tract cancer on basis of the calculated concentration
wherein the marker comprises Nudifloramide.

10. The apparatus of claim 9, wherein the marker further comprises at least one of LPC 18:0 and fibrinogen alpha chain.

11. The apparatus of claim 10, criterion concentration of the Nudifloramide for biliary tract cancer diagnosis is 220 pg/µℓ to 320 pg/µℓ.

## Patentansprüche

1. Verfahren zur Bereitstellung diagnostischer Informationen zwecks Diagnose von Gallenwegskrebs, umfassend:
Messen einer Konzentration eines Markers zum Vorhersagen von Gallenwegskrebs in zuvor erhaltenen biologischen Proben, welche Vollblut einschließen; und
Bereitstellen diagnostischer Informationen in Bezug auf Gallenwegskrebs mittels der gemessenen Konzentration des Markers,
wobei der Marker Nudifloramid umfasst.

2. Verfahren nach Anspruch 1, wobei der Marker ferner mindestens eines von LPC 18:0 und einer Fibrinogen alpha-Kette umfasst.

3. Verfahren nach Anspruch 1 oder Anspruch 2, wobei eine Kriteriumskonzentration des Nudifloramids für eine Gallenwegskrebsdiagnose 220 pg/µl bis 320 pg/µl beträgt.

4. Verfahren nach Anspruch 2, wobei eine Kriteriumskonzentration der Fibrinogen alpha-Kette 130 pg/µl bis 200 pg/µl beträgt.

5. Verfahren nach Anspruch 2, wobei die Konzentration des LPC 18:0 mittels eines Massenspektrometers gemessen wird, und die Kriteriumskonzentration des LPC 18:0 für eine Gallenwegskrebsdiagnose 1 500 000 au bis 2 000 000 au beträgt.

6. Verfahren nach Anspruch 2, wobei die Bereitstellung der Informationen die Ermittlung dessen ist, ob Gallenwegskrebs vorliegt oder nicht, auf Grundlage einer bestimmten Konzentration des Nudifloramids und der Fibrinogen alpha-Kette oder mehr und einer bestimmten Konzentration des LPC 18:0 oder weniger ist.

7. Verfahren nach Anspruch 1, wobei die biologischen Proben ferner Serum umfassen.

8. Verfahren nach Anspruch 1, wobei die Konzentration durch Massenspektrometrie gemessen wird.

9. Gerät zum Diagnostizieren von Gallenwegskrebs, umfassend:
eine Eingabeeinheit, konfiguriert zum Eingeben von aus biologischen Proben erfassten Massenspektrumsdaten; und
eine Diagnoseeinheit, konfiguriert zum Berechnen einer Konzentration eines Markers zur Vorhersage von Gallenwegskrebs aus den Massenspektrumsdaten und Ermitteln diagnostischer Informationen in Bezug auf Gallenwegskrebs auf Grundlage der berechneten Konzentration,
wobei der Marker Nudifloramid umfasst.

10. Gerät nach Anspruch 9, wobei der Marker ferner mindestens eines von LPC 18:0 und einer Fibrinogen alpha-Kette umfasst.

11. Gerät nach Anspruch 10, wobei eine Kriteriumskonzentration des Nudifloramids für eine Gallenwegskrebsdiagnose 220 pg/µl bis 320 pg/µl beträgt.

## Revendications

1. Procédé pour la fourniture d'informations de diagnostic pour le diagnostic d'un cancer du tractus biliaire comprenant:
la mesure de la concentration d'un marqueur pour prévoir un cancer du tractus biliaire dans des échantillons biologiques obtenus précédemment incluant du sang total; et
la fourniture d'informations de diagnostic pour un cancer du tractus biliaire en utilisant la concentration mesurée du marqueur,
dans lequel le marqueur comprend le Nudifloramide.

2. Procédé selon la revendication 1, dans lequel le marqueur comprend en outre au moins un parmi LPC 18:0 et une chaîne alpha du fibrinogène.

3. Procédé selon la revendication 1 ou la revendication 2, dans lequel la concentration critère du Nudifloramide pour un cancer du tractus biliaire est de 220 pg/µl à 320 pg/µl.

4. Procédé selon la revendication 2, dans lequel la concentration critère de la chaîne alpha du fibrinogène est de 130 pg/µl à 200 pg/µl.

5. Procédé selon la revendication 2, dans lequel la concentration du LPC 18:0 est mesurée en utilisant un spectromètre de masse et la concentration critère du LPC 18:0 pour un diagnostic de cancer du tractus biliaire est de 1.500.000 au à 2.000.000 au.

6. Procédé selon la revendication 2, la fourniture des informations est une détermination de si un cancer du tractus biliaire est présent ou non sur la base d'une certaine concentration du Nudifloramide et de la chaîne alpha du fibrinogène ou plus et une certaine concentration du LPC 18:0 ou moins.

7. Procédé selon la revendication 1, dans lequel les échantillons biologiques comprennent en outre un sérum.

8. Procédé selon la revendication 1, dans lequel la concentration est mesurée par une spectrométrie de masse.

9. Appareil pour le diagnostic d'un cancer du tractus biliaire comprenant:
une unité d'entrée configurée pour entrer les données de spectre de masse détectées à partir d'échantillons biologiques; et
une unité de diagnostic configurée pour calculer la concentration d'un marqueur pour prévoir un cancer du tractus biliaire à partir des données de spectre de masse et déterminer les informations de diagnostic pour un cancer du tractus biliaire sur la base de la concentration calculée,
dans lequel le marqueur comprend le Nudifloramide.

10. Appareil selon la revendication 9, dans lequel le marqueur comprend en outre au moins un parmi LPC 18:0 et une chaîne alpha du fibrinogène.

11. Appareil selon la revendication 10, dans lequel une concentration critère du Nudifloramide pour un cancer du tractus biliaire est de 220 pg/µl à 320 pg/µl.
